(19) Europäisches Patentamt
European Patent Office
Office européen des brevets

(11) Publication number: **0 117 368 A1**

(12) **EUROPEAN PATENT APPLICATION**

(21) Application number: **83308037.7**

(22) Date of filing: **30.12.83**

(51) Int. Cl.³: **C 07 D 417/12**, C 07 D 403/12, C 07 D 257/04, C 07 D 405/12, A 61 K 31/33

(30) Priority: **31.12.82 GB 8237069**
**24.03.83 GB 8308171**
**19.04.83 GB 8310519**

(43) Date of publication of application: **05.09.84**
**Bulletin 84/36**

(84) Designated Contracting States: **AT BE CH DE FR GB IT LI LU NL SE**

(71) Applicant: **GLAXO GROUP LIMITED, Clarges House 6-12 Clarges Street, London W1Y 8DH (GB)**

(72) Inventor: **Bays, David Edmund, 9 Windmill Field Ware, Hertfordshire (GB)**
Inventor: **Hayes, Roger, 5 Sandringham Road Potters Bar, Hertfordshire (GB)**
Inventor: **Blatcher, Philip, 43 Trafalgar Avenue Broxbourne, Hertfordshire (GB)**
Inventor: **Atkinson, Michael, 52 The Ridgeway Ware, Hertfordshire (GB)**

(74) Representative: **Marchant, James Ian et al, Elkington and Fife High Holborn House 52/54 High Holborn, London WC1V 6SH (GB)**

(54) **Guanidinoazolyl derivatives as histamine H2 antagonists.**

(57) This invention relates to compounds of the general formula (I)

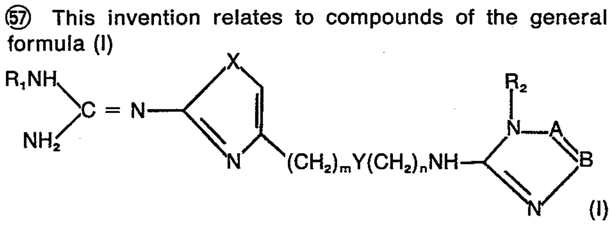

(I)

and physiologically acceptable salts and hydrates thereof, in which

$R_1$ represents a hydrogen atom or an alkyl, alkanoyl, aroyl or trifluoroalkyl group;

$R_2$ represents a hydrogen atom or an alkyl or alkenyl group or $C_{2-6}$ alkyl group substituted by a hydroxy or alkoxy group;

X represents a sulphur atom or NH;

Y represents an oxygen or sulphur atom or a bond;

m represents 1, 2 or 3;

n represents 2, 3 or 4;

A represents N and B represents $CR_3$; or

A represents $CR_3$ and B represents N; or

A and B each represent N; and

$R_3$ represents a hydroxy, hydroxyalkyl, alkoxyalkyl, acyloxyalkyl or CH=NOH group; or

$R_3$ represents the group $CH_2Z(CH_2)_rR_4$ in which r is 1 to 4 when Z is an oxygen atom, or r is 1 when Z is a sulphur atom, and $R_4$ is a $-CH_2OH$ group, the group $-CH=CH_2$, the group $-CH_2NR_5R_6$ where $R_5$ and $R_6$ are each alkyl or the group $-CH_2NHR_7$ where $R_7$ is alkyl; or

$R_3$ represents an aralkyl or heteroalkyl group in which the alkyl portion is optionally substituted by a hydroxy or acyloxy group; or

$R_3$ represents the group $(CH_2)_qR_8$ where q is zero, 1, 2 or 3 and $R_8$ is the group $CH_2NHC(=D)NHR_9$ where D is NCN or $CHNO_2$ and $R_9$ is an alkyl group; or the group $COR_{10}$ where $R_{10}$ is a hydrogen atom or a hydroxy, alkyl or $NR_{11}R_{12}$ group where $R_{11}$ and $R_{12}$ are the same or different and each represents a hydrogen atom or an alkyl group, or $NR_{11}R_{12}$ forms a 5 or 6 membered ring; or the group $SO_2R_{13}$ where $R_{13}$ is an alkyl or aryl group; or the group $NHSO_2R_{14}$ where $R_{14}$ is an alkyl group; or the group $NHCOR_{15}$ where $R_{15}$ is a hydrogen atom or an alkyl, alkoxy, aryl, heteroaryl or $NHR_{16}$ group where $R_{16}$ is an aryl, alkyl or cycloalkyl group; or

$R_3$ represents the group $CH_2SR_{17}$ where $R_{17}$ is an alkyl or heteroaryl group; or

$R_3$ represents a $C_{2-4}$ straight or branched alkyl group substituted by two hydroxyl groups or the dihydroxyalkyl group may form a cyclic ketal structure of the formula

(Continuation next page)

EP 0 117 368 A1

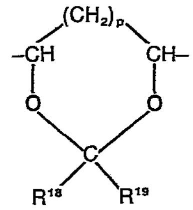

where p is zero or 1, and $R_{18}$ and $R_{19}$, which may be the same or different, each represents a $C_{1-4}$ alkyl group.

The compounds show pharmacological activity as selective histamine $H_2$-antagonists.

TITLE MODIFIED
see front page



HETEROCYCLIC DERIVATIVES

This invention relates to novel heterocyclic derivatives having action on histamine receptors, to processes for the preparation thereof, to pharmaceutical compositions containing them and to their use in therapeutics.

Certain novel heterocyclic derivatives have now been found which have potent activity as $H_2$-antagonists. These compounds which are more particularly described below, for example show inhibition of the secretion of gastric acid when this is stimulated via histamine receptors (Ash and Schild, Brit. J. Pharmacol. Chemother. 1966, 27, 427). Their ability to do so can be demonstrated in the perfused rat stomach using the method described in British Patent Specification No. 1,565,966, modified by the use of sodium pentobarbitone (50 mg/kg) as anaesthetic instead of urethane, and in conscious dogs equipped with Heidenhain pouches using the method described by Black et al Nature 1972 236, 385. Furthermore, the compounds antagonise the effect of histamine on the contraction frequency of isolated guinea pig right atrium. Certain compounds according to the invention have the advantage of an extended duration of action.

Compounds with histamine $H_2$-blocking activity may be used in the treatment of conditions where there is an advantage in lowering gastric acidity, particularly in

gastric and peptic ulceration, as a prophylactic measure in surgical procedures, and in the treatment of allergic and inflammatory conditions where histamine is a known mediator. Thus they may be used for example, either alone, or in combination with other active ingredients in the treatment of allergic and inflammatory conditions of the skin.

The present invention provides compounds of the general formula (I)

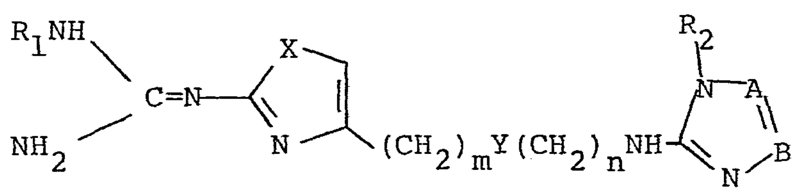

(I)

and physiologically acceptable salts and hydrates thereof, in which

$R_1$ represents a hydrogen atom or an alkyl, alkanoyl, aroyl or trifluoroalkyl group;

$R_2$ represents a hydrogen atom or an alkyl or alkenyl group or $C_{2-6}$ alkyl group substituted by a hydroxy or alkoxy group,

X represents a sulphur atom or NH;

Y represents an oxygen or sulphur atom or a bond;

m represents 1, 2 or 3;

n represents 2, 3 or 4;

A represents N and B represents $CR_3$; or

A represents $CR_3$ and B represents N; or A and B each represent N; and

$R_3$ represents a hydroxy, hydroxyalkyl, alkoxyalkyl, acyloxylalkyl or CH=NOH group; or

$R_3$ represents the group $CH_2Z(CH_2)_rR_4$ in which r is 1 to 4 when Z is an oxygen atom, or r is 1 when Z is a sulphur atom, and $R_4$ is a $-CH_2OH$ group, the group $-CH=CH_2$, the group $-CH_2NR_5R_6$ where $R_5$ and $R_6$ are each alkyl or the group $-CH_2NHR_7$ where $R_7$ is alkyl; or

$R_3$ represents an aralkyl or heteroaralkyl group in which the alkyl portion is optionally substituted by a hydroxy or acyloxy group; or

$R_3$ represents the group $(CH_2)_qR_8$ where q is zero, 1, 2 or 3 and $R_8$ is the group $CH_2NHC(=D)NHR_9$ where D is NCN or $CHNO_2$ and $R_9$ is an alkyl group; or the group $COR_{10}$ where $R_{10}$ is a hydrogen atom or a hydroxy, alkyl or $NR_{11}R_{12}$ group where $R_{11}$ and $R_{12}$ are the same or different and each represents a hydrogen atom or an alkyl group, or $NR_{11}R_{12}$ forms a 5 or 6 membered ring; or the group $SO_2R_{13}$ where $R_{13}$ is an alkyl or aryl group; or the group $NHSO_2R_{14}$ where $R_{14}$ is an alkyl group; or the group $NHCOR_{15}$ where $R_{15}$ is a hydrogen atom or an alkyl, alkoxy, aryl, heteroaryl or $NHR_{16}$ group where $R_{16}$ is an aryl, alkyl or cycloalkyl group; or

$R_3$ represents the group $CH_2SR_{17}$ where $R_{17}$ is an alkyl or heteroaryl group; or

$R_3$ represents a $C_{2-4}$ straight or branched alkyl group substituted by two hydroxyl groups or the dihydroxyalkyl group may form a cyclic ketal structure of the formula

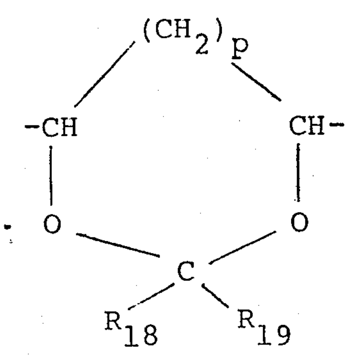

where p is zero or 1, and $R_{18}$ and $R_{19}$, which may be the same or different, each represents a $C_{1-4}$ alkyl group.

In the above formula (I) the term 'alkyl' as a group or part of a group means that the group is straight or branched and, unless otherwise stated, contains 1 to 6 carbon atoms, and in particular 1 to 4 carbon atoms,

e.g. methyl or ethyl, and the term 'alkenyl' means that the group has preferably 3 to 6 carbon atoms. The term 'cycloalkyl' means that the group has 3 to 8 carbon atoms. The term 'aryl' as a group or part of a group preferably means phenyl or substituted phenyl, for example phenyl substituted with one or more $C_{1-3}$ alkyl or $C_{1-3}$ alkoxy groups, or halogen atoms, e.g. flourine. The term 'acyl' means an aroyl, aralkanoyl or $C_{1-6}$ alkanoyl group, e.g. acetyl, formyl, phenylacetyl or benzoyl. The term 'heteroaryl' within the definition of $R_3$ as an optionally substituted heteroaralkyl group or within the definition of $R_{15}$ means a 5 to 6 membered monocyclic ring containing one heteroatom selected from oxygen, nitrogen and sulphur, e.g. thienyl, pyridinyl or furyl, optionally substituted by a $C_{1-3}$ alkyl group. The alkyl portion of a heteroaralkyl group is a straight or branched $C_{1-4}$ alkyl chain, and the heteroaryl ring is linked to the alkyl portion through a carbon atom. The term 'heteroaryl' within the definition of $R_{17}$ means tetrazolyl, triazolyl or thiadiazolyl optionally substituted by $C_{1-3}$ alkyl (e.g. 1-methyltetrazol-5-yl).

According to one aspect the invention relates to compounds of formula (I) in which A and B each represent N and $R_1$ is other than a trifluoroalkyl group.

According to another aspect the invention relates to compounds of formula (I) in which A represents N and B represents $CR_3$ or A represents $CR_3$ and B represents N; m is 1 or 2 and n is 2 or 3; and $R_3$ is other than the group $CH_2Z(CH_2)_rR_4$ in which Z is an oxygen atom and r is 4, or Z is a sulphur atom and $R_4$ is a $-CH_2OH$, $-CH_2NR_5R_6$ or $-CH_2NHR_7$ group.

The following are examples of suitable meanings for the groups $R_1$, $R_2$ and $R_3$:

$R_1$ may be for example a hydrogen atom or a methyl, ethyl, propyl, isopropyl, butyl, acetyl, propionyl, benzoyl or 2,2,2-trifluoroethyl group.

$R_2$ may be for example a methyl, ethyl, propyl, allyl, 2-hydroxyethyl or 2-methoxyethyl group.

$R_3$ may be for example a group selected from hydroxy; hydroxymethyl; methoxymethyl; acetoxymethyl; formyloxymethyl; benzoyloxymethyl; phenacetoxymethyl; -CH=NOH; $-CH_2Z(CH_2)_rR_4$ where $R_4$ is hydroxy, $-CH=CH_2$, di $C_{1-3}$ alkylamino (e.g. dimethylamino) or $NHR_7$ where $R_7$ is $C_{1-3}$ alkyl (e.g. methyl); phenyl $C_{1-3}$ alkyl (e.g. benzyl) or heteroaryl $C_{1-3}$ alkyl (e.g. furylmethyl or pyridylmethyl) in which the alkyl portion in each is unsubstituted or substituted by -OH or $C_{1-4}$ alkanoyloxy (e.g. acetyloxy); methylthiomethyl; $(CH_2)_qR_8$, where $R_8$ is $CH_2NHC(=D)NHR_9$ (where D is NCN or $CHNO_2$ and $R_9$ is methyl); CHO; $-CO_2H$; alkoxycarbonyl (e.g. methoxycarbonyl); $-CONH_2$; $-CONHCH_3$; $-CON(CH_3)_2$; $CONR_{11}R_{12}$ (where $NR_{11}R_{12}$ is pyrrolidino); $SO_2R_{13}$ where $R_{13}$ is methyl or phenyl; $-NHSO_2CH_3$; NHCHO; $NHCOR_{15}$ where $R_{15}$ is methyl, ethoxy or phenyl; $NHCONHR_{16}$ where $R_{16}$ is phenyl, methyl or $C_{3-8}$ cycloalkyl; or 2,2-di-$C_{1-3}$ alkyl (e.g. dimethyl)-1,3-dioxolan-4-yl.

Examples of suitable chains $-(CH_2)_mY(CH_2)_n-$ are $-CH_2S(CH_2)_2-$, $-CH_2O(CH_2)_3$ and $-(CH_2)_4-$.

Within the definition of formula (I)

$R_1$ is preferably a hydrogen atom;

$R_2$ is preferably a hydrogen atom, a $C_{1-4}$ alkyl group (e.g. methyl) or a $C_{2-6}$ alkyl group substituted by a hydroxy group (e.g. 2-hydroxyethyl);

X is preferably a sulphur atom;

Y is preferably a sulphur atom;

m is preferably 1;

n is preferably 2;

$R_3$ is preferably a hydroxy $C_{1-3}$ alkyl group (e.g. hydroxymethyl or hydroxyethyl, particularly hydroxymethyl).

Particularly preferred compounds according to the invention are N-[4-[[[2-[[3-(hydroxymethyl)-1-methyl-1_H_-1,2,4-triazol-5-yl]amino]ethyl]thio]methyl]-2-thiazolyl] guanidine,

and physiologically acceptable salts thereof; and

N-[4-[[[2-[(1-methyl-1H-tetrazol-5-yl)amino]ethyl]thio]methyl]-2-thiazolyl]guanidine,
and physiologically acceptable salts thereof; and
N-[4-[[[2-[[1-(2-hydroxyethyl)-1H-tetrazol-5-yl]amino]ethyl]thio]methyl]-2-thiazolyl]guanidine,
and physiologically acceptable salts thereof.

The invention includes the compounds of formula (I) in the form of physiologically acceptable salts with inorganic and organic acids. Particularly useful salts include hydrochlorides, hydrobromides, sulphates, methanesulphonates, acetates, maleates, succinates, citrates, tartrates, fumarates and benzoates. The compounds of formula (I) and their salts may also form hydrates, which hydrates are also to be considered as part of the invention. The compounds of formula (I) can exhibit tautomerism and the formula is intended to cover all tautomers. Where optical isomers may exist the formula is intended to cover all diastereoisomers and optical enantiomers. The present invention also extends to bioprecursors of the compounds of formula (I). Bioprecursors are compounds which have a structure different to that of the compounds of formula (I) but which, upon administration to the animal or human being are converted in the body into a compound of formula (I).

The compounds according to the invention, preferably in the form of a salt, may be formulated for administration in any convenient way and the invention includes within its scope pharmaceutical compositions containing at least one compound according to the invention adapted for use in human or veterinary medicine. Such compositions may be formulated in a conventional manner using one or more pharmaceutically acceptable carriers or excipients. Such compositions may also contain if required other active ingredients, e.g. $H_1$-antagonists.

Thus the compounds according to the invention may be formulated for oral, buccal, topical, parenteral or rectal administration. Oral administration is preferred.

For oral administration, the pharmaceutical composition may take the form of for example, tablets, capsules, powders, solutions, syrups or suspensions prepared by conventional means with acceptable excipients. For buccal administration the composition may take the

the form of tablets or lozenges formulated in conventional manner.

The compounds of the invention may be formulated for parenteral administration by bolus injection or continuous infusion. Formulations for injection may be presented in unit dosage form in ampoules, or in multidose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for reconstitution with a suitable vehicle e.g. sterile pyrogen-free water before use.

The compounds of the invention may also be formulated in rectal compositions such as suppositories or retention enemas, e.g. containing conventional suppository bases such as cocoa butter or other glyceride.

For topical application, the compounds of the invention may be formulated as ointments, creams, gels, lotions, powders or sprays in a conventional manner.

For internal administration a convenient daily dosage regime of the compounds according to the invention is 1 to 4 doses to a total of 5 mg to 1 g per day, preferably 5 to 500 mg per day, dependent upon the condition of the patient.

It will be appreciated that in the methods for the preparation of compounds of formula (I) given below, for certain reaction steps it may be necessary to protect various reactive substituents in the starting materials for a particular reaction and subsequently to remove the protecting group. Such protection and subsequent deprotection may be particularly pertinent where $R_2$ is an alkyl group bearing a hydroxy substituent and/or when $R_3$ contains a hydroxy or amino group. Standard protection and deprotection procedures can be employed, for example, amines may be protected by formation of a phthalimide

group which may subsequently be cleaved by treatment with a hydrazine, e.g. hydrazine hydrate or a primary amine, for example methylamine. Hydroxyl groups may for example be protected by formation of ethers e.g. tetrahydropyranyl, or esters of carboxylic acids such as alkanoic acids, e.g. acetic acid, which may subsequently be removed by hydrolysis.

In describing the processes which may be used for preparing the compounds of formula (I) or intermediates useful in the preparation thereof, any of $R_1$ to $R_{19}$, A, B, D, Alk, X, Y, Z, n, m, p, q and r in the various formulae are as defined in formula (I) unless otherwise stated.

Compounds of formula (I) in which A or B is $-CR_3$ may be prepared by cyclisation of an appropriate intermediate. Thus compounds of formula (I) in which $R_3$ is other than a dihydroxyalkyl group forming a cyclic acetal or cyclic ketal structure, or an aralkyl or heteroaralkyl group in which the alkyl portion is substituted by an acyloxy group, or acyloxyalkyl, CH=NOH, $SO_2R_{13}$, $COR_{10}$ (where $R_{10}$ is hydrogen or alkyl), or the group $(CH_2)_qR_8$, where $R_8$ is $CH_2NHC(=D)NHR_9$ can be prepared by cyclisation of a compound of formula (II)

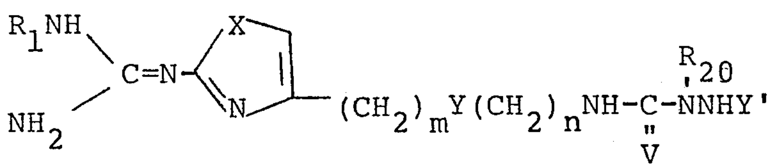

(II)

in which $R_{20}$ is a group as defined for $R_2$, V' is $N\underset{\underset{V}{\|}}{C}R_3^{a}$ and Y' is hydrogen where V is oxygen or sulphur and $R_3^{a}$ is a group as defined for $R_3$ or a group convertible thereto under the conditions of the cyclisation reaction; or V' is $NR_2$, $R_{20}$ is hydrogen and Y' is $\underset{\underset{Y''}{\|}}{C}R_3^{a}$ where Y" is oxygen or sulphur.

Thus for example in an embodiment of the cyclisation process a compound of formula (I) in which A is N and B is

the group $CR_3$ may be prepared by cyclisation of a compound of formula (III)

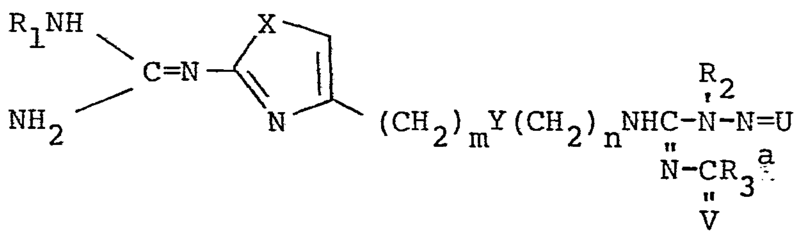

(III)

where V represents sulphur or more preferably oxygen and U represents two hydrogen atoms, in the absence or presence of a solvent, e.g. a hydrocarbon such as toluene, a ketone such as acetone, an alkanol such as methanol, or water, and optionally with heating, for example, within the range 50° to 90°.

It may be convenient to prepare in situ compounds of formula (III) in which U represents two hydrogen atoms by treating a compound of formula (III) where U represents a divalent protecting group which can readily be removed to yield two hydrogen atoms, for example a benzylidene group, with an acid, e.g. hydrochloric acid, preferably with heating, and under such conditions cyclisation to give the corresponding compound of formula (I) will normally occur.

In general intermediates of formula (III) may be prepared from the diamines of formula (IV)

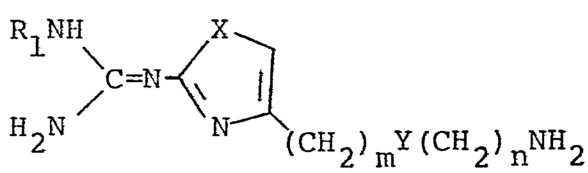

(IV)

by methods analogous to those described in British Patent Specification No. 2047238A.

Compounds of formula (I) in which A and B both represent N may be prepared by reacting an amine of formula (IV)

preferably in the form of a salt, e.g. a hydrochloride, with a tetrazole of formula (V)

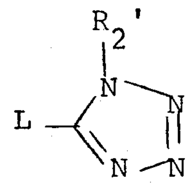

(V)

where L is a leaving group such as halogen, e.g. bromine, and $R_2'$ is the group $R_2$ or a group convertible thereto. The reaction is preferably carried out with heating, for example within the range 80 to 200°C, in the absence or presence of a solvent such as an alkanol, e.g. butanol, optionally in a sealed vessel and preferably in the presence of a base such as triethylamine.

The compounds of formula (IV) may be prepared as described in British Patent Specification No. 2001624A. The tetrazoles of formula (V) in which L represents a leaving group such as halogen are either known compounds or may be prepared by methods analogous to those described in British Patent Specification No. 1364917 and G.B. Barlin, J. Chem. Soc., (B), 1967, 641, e.g. by halogenation e.g. bromination of a compound of formula (VI)

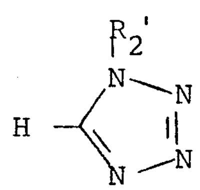

(VI)

Tetrazoles of formula (VI) are either known compounds or, when $R_2'$ is a hydroxyalkyl group or a group convertible thereto, may be prepared by reaction of a corresponding aminoalkanol derivative $NH_2-R_2'$ with sodium azide and triethylorthoformate in the presence of a solvent such as acetic acid.

Compounds of formula (V) in which L is a leaving group such as halogen, e.g. bromine or chlorine and $R_2'$ is the group $CH_2CH_2OR_{20}$ where $R_{20}$ is a hydrogen atom or

a hydroxyl protecting group, e.g. tetrahydropyranyl ether, are novel compounds and represent a further aspect of the present invention.

Compounds of formula (I) in which $R_3$ is the group $(CH_2)_qR_8$ in which $R_8$ is $COR_{10}$ where $R_{10}$ is hydrogen or alkyl, or $SO_2R_{13}$ may be prepared by oxidation of the corresponding compound in which $R_3$ is the group $(CH_2)_qCHR_{10}OH$ or $(CH_2)_qSR_{13}$.

Thus aldehydes and ketones of formula (I) in which $R_3$ is the group $(CH_2)_qCOR_{10}$ where $R_{10}$ is hydrogen or alkyl

may be prepared by oxidising the corresponding hydroxyalkyl compounds in which $R_3$ is $(CH_2)_qCHR_{10}OH$ using for example oxalyl chloride and dimethylsulphoxide, or activated manganese dioxide in a solvent such as dichloromethane.

Compounds of formula (I) in which $R_3$ is the group $(CH_2)_qSO_2R_{13}$ may be prepared by oxidising the corresponding compound in which $R_3$ is $-(CH_2)_qSR_{13}$ with for example peracetic acid or nitric acid. The reaction may be carried out in a solvent such as acetic acid, at room temperature.

Starting materials in which $R_3$ represents $-SR_{13}$ may be prepared from the corresponding compound in which $R_3$ is -SH. These intermediates may in turn be prepared by cyclisation of a compound of formula (III) in which V represents sulphur and $R_3{}^a$ represents halogen e.g. chlorine.

Compounds of formula (I) in which $R_3$ is CH=NOH may be prepared by reacting the corresponding aldehyde with hydroxylamine in a suitable solvent such as ethanol, optionally with heating.

Compounds of formula (I) in which $R_3$ is acyloxyalkyl, or $R_3$ represents an aralkyl or heteroaralkyl group in which the alkyl portion is substituted by an acyloxy group may be prepared by reacting the corresponding alcohol with an activated derivative (e.g. an acid chloride or an acid anhydride) of an appropriate acid. The reaction may be carried out at room temperature, optionally in the presence of a solvent (e.g. pyridine, tetrahydrofuran, acetone or dimethylformamide), and preferably in the presence of a base (e.g. pyridine, triethylamine or an alkali metal carbonate such as potassium carbonate).

Compounds of formula (I) in which $R_3$ includes the group $-\underset{OH}{CH}(CH_2)_p\underset{OH}{CH}-$ may be converted into the corresponding compounds in which $R_3$ includes the group

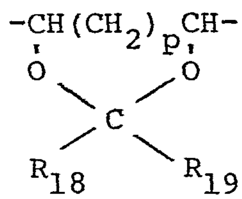 by reaction with a ketone $R_{18}R_{19}CO$, e.g. acetone in the presence of an acid, e.g. p-toluenesulphonic acid. The reaction is carried out in the absence or presence of a solvent, e.g. benzene, at a temperature between room temperature and reflux.

Compounds of formula (I) in which $R_3$ represents the group $CH_2NHC(=D)NHR_9$ may be prepared by treating the corresponding aminoalkyltriazole in which $R_3$ is the group $(CH_2)_{q+1}NH_2$ with a compound of formula $LC(=D)NHR_9$, where L is a leaving group (e.g. methylthio). The reactants may be mixed for example in an aqueous solution at room temperature.

Where the product of any one of the above processes is a free base and an acid addition salt, in particular a physiologically acceptable salt is required, the salt may be formed in conventional manner. Thus, for example, a generally convenient method of forming the salts is to mix appropriate quantities of the free base and the acid in an appropriate solvent(s) e.g. an alcohol such as ethanol or an ester such as ethyl acetate. The invention also includes interconversion of one salt of the compound of formula (I) into another.

The invention is illustrated but not limited by the following Examples and Preparations, in which temperatures are in °C.

Preparation 1

1H-Tetrazole-1-ethanol acetate (ester)

2-Aminoethanol acetate (ester) hydrochloride (4.19 g), sodium azide (2.34 g), triethylorthoformate (6.67 g) and acetic acid (6 ml) were stirred at 70° for 24 h. The mixture was cooled and acidified to pH 1 with concentrated

hydrochloric acid. After 2h, excess saturated potassium carbonate solution was added and the mixture was extracted with ethyl acetate to give the title compound (4g) as a pale yellow oil.

N.m.r. (DMSO): 0.49, s, (1H); 5.23, s, (2H); 5.57, t, (2H); 8.00, s, (3H).

Preparation 2

5-Bromo-1H-tetrazole-1-ethanol acetate (ester)

Bromine (69.1g) in chloroform (80 ml) was added to a stirred refluxing solution of 1H-tetrazole-1-ethanol acetate (ester) (33.8 g) in acetic acid (200 ml) and chloroform (400 ml). After 72 h the mixture was cooled and evaporated, excess, saturated potassium carbonate solution was added and the mixture was extracted with ethyl acetate to give the title compound (48.2 g) as a cream solid, m.p. 55-6° (from diethyl ether).

Preparation 3

5-Bromo-1H-tetrazole-1-ethanol

5-Bromo-1H-tetrazole-1-ethanol acetate (ester) (40 g) and 2N hydrochloric acid (216 ml) were stirred at room temperature for 22h.

The solution was concentrated to ca. 75 ml and basified with excess solid potassium carbonate. Any solid present was dissolved by the addition of water and the solution was extracted with ethyl acetate.

The combined extracts were dried and evaporated to give a yellow oil which solidified. Recrystallisation from isopropyl acetate gave the title compound (24.7 g) as a white solid m.p. 73-4°.

Preparation 4

5-Bromo-1-[2-[(tetrahydro-2H-pyran-2-yl)oxy]ethyl]-1H-tetrazole

A suspension of 5-bromo-1H-tetrazole-1-ethanol (5.79 g)

and pyridinium 4-toluenesulphonate (0.5 g) in dichloromethane (50 ml) and dihydropyran (4 ml) was stirred at room temperature for 16 h to give a colourless solution. Water (25 ml) and sodium carbonate solution (25 ml) were added, the phases were separated and the aqueous phase was extracted with dichloromethane. The combined extracts were washed with water and brine, dried and evaporated to give the title compound (6.5 g) as a colourless oil.

| | | | |
|---|---|---|---|
| Assay Found: | C, 35.1; | H, 4.81; | N, 20.0. |
| $C_8H_{13}BrN_4O_2$ requires | C, 34.7; | H, 4.73; | N. 20.2% |

Example 1

N-[4-[[[2-[[3-(Hydroxymethyl)-1-methyl-1H-1,2,4-triazol-5-yl]amino]ethyl]thio]methyl]-2-thiazolyl]guanidine difumarate

A solution of N-[4-[[(2-aminoethyl)thio]methyl]-2-thiazolyl]guanidine, dihydrochloride (1.53 g) and triethylamine (1.01 g) in methanol (30 ml) was stirred at room temperature for 0.5h, and methyl N-[2-(acetyloxy)acetyl]-1-methyl-2-(phenylmethylene)-hydrazine carboximidothioate (1.69 g) in methanol (20 ml) was added. The mixture was stirred at room temperature for 18h and evaporated in vacuo. The residue was stirred with 5M hydrochloric acid (25 ml) and toluene (50 ml) for 16 h. The two

phases were separated, and the acidic aqueous layer was basified to pH 6 with sodium carbonate, and washed with toluene. The aqueous layer was then basified with excess sodium carbonate and extracted with ethyl acetate. The organic extract was dried ($Na_2SO_4$) and evaporated to leave a brown gum which was chromatographed on a column of alumina using dichloromethane:ethanol:ammonia (25:8:1) to give a foam which was dissolved in a mixture of ethanol and acetone and treated with a solution of fumaric acid in acetone to precipitate a white solid. This solid was recrystallised from absolute ethanol to give the title compound (0.11 g) as a white solid m.p. 165°.

| | | | |
|---|---|---|---|
| Assay Found: | C, 39.3; | H, 4.8; | N, 19.9; |
| $C_{11}H_{18}N_8S_2O.2C_4H_4O_4$ requires: | C, 39.7; | H, 4.5; | N, 19.5% |

Example 2

N-[4-[[[2-[(1-Methyl-1H-tetrazol-5-yl)amino]ethyl]thio]methyl]-2-thiazolyl]guanidine

A solution of N-[4-[[(2-aminoethyl)thio]methyl]-2-thiazolyl]guanidine dihydrochloride (1.53 g) and triethylamine (2.5 ml) in n-butanol (45 ml) was stirred at 20° for 0.5 hours and then 5-bromo-1-methyl-1H-tetrazole (0.82 g) was added. The mixture was heated at reflux for 24 hours and the solvent removed *in vacuo*. The dark brown residue was partitioned between ethyl acetate and aqueous sodium carbonate. The ethyl acetate extract was washed with brine and evaporated to leave a brown gum which was chromatographed on silica using methanol: ammonia (250:1) to give a dark brown gum (0.6g). This gum was chromatographed on alumina using dichloromethane: ethanol:0.88 ammonia (100:8:1) to give a brown solid (0.16 g) which was crystallised from acetone to give the title compound (0.038 g) as a cream white solid m.p. 80-3°. N.m.r. ($CD_3OD$): 3.46, s, (1H); 6.25, s, (3H); 6.32, s, (2H); 6.44, t, (2H); 7.23, t, (2H).

Example 3

N-[4-[[[2-[[1-(2-Hydroxyethyl)-1H-tetrazol-5-yl]amino]ethyl]thio]methyl]-2-thiazolyl]guanidine, d,ℓ-tartarate

A solution of N-[4-[[(2-aminoethyl)thio]methyl]-2-thiazolyl]guanidine, dihydrochloride (1.3 g) and triethylamine (1.3 ml) in butan-1-ol (10 ml) was stirred under nitrogen for 1h, and 5-bromo-1-[2-[tetrahydro-2H-pyran-2-yl]oxy]ethyl]-1H-tetrazole (1.2 g) in butan-1-ol (5 ml) was added. The reaction solution was gently refluxed for 26h and evaporated *in vacuo* to leave a black solid residue which was chromatographed on a column of alumina using dichloromethane:ethanol:ammonia - (150:8:1), to give a brown gum. This was dissolved in dilute hydrochloric acid, stirred for 2h at room temperature and washed with diethyl ether. The pH of the aqueous phase was adjusted to pH 8 with sodium carbonate and extracted with diethyl ether. The organic extract was dried ($MgSO_4$) and evaporated to leave a light brown foam. This foam was chromatographed on a column of silica using dichloromethane:ethanol:ammonia -(50:8:1) to give a colourless gum (0.1 g) which was dissolved in absolute ethanol and added to a solution of d,ℓ-tartaric acid in absolute ethanol. The resulting solution was stirred for 2h and dry diethyl ether was added to precipitate the *title* compound (0.105 g) as a white hygroscopic solid m.p. 60-65°.

N.m.r. ($D_2O$): 3.00, s, (1H); 5.62, s, (2H); 5.72, t, (2H); 6.06, t, (2H); 6.20, s, (2H); 6.52, t, (2H); 7.15, t, (2H).

Examples of Pharmaceutical Compositions

Tablets

| | mg/tablet |
|---|---|
| Active ingredient | 20.0 |
| Microcrystalline Cellulose USP | 178.5 |
| Magnesium Stearate BP | 1.5 |
| Compression weight | 200.0 |

The active ingredient is sieved through a suitable sieve, blended with the excipients and compressed using 7 mm diameter punches.

Tablets of other strengths may be prepared by altering the compression weight and using punches to suit.

The tablets may be film coated with suitable film forming materials, such as hydroxypropyl methylcellulose, using standard techniques. Alternatively the tablets may be sugar coated.

Injection for Intravenous Administration

| | % w/v |
|---|---|
| Active ingredient | 0.2 |
| Sodium Chloride BP | as required |
| Water for Injection BP to | 100.00 |

Sodium chloride may be added to adjust the tonicity of the solution and the pH may be adjusted, using acid or alkali, to that of optimum stability and/or facilitate solution of the active ingredient. Alternatively suitable buffer salts may be used.

The solution is prepared, clarified and filled into appropriate size ampoules sealed by fusion of the glass. The injection is sterilised by heating in an autoclave using one of the acceptable cycles. Alternatively the solution may be sterilised by filtration and filled into sterile ampoules under aseptic conditions. The solution

may be packed under an inert atmosphere of nitrogen or another suitable gas.

The ability of the compounds of the invention to inhibit histamine induced gastric acid was demonstrated in the perfused rat stomach test using the method described in British Patent Specification No. 1565966 modified by the use of sodium pentobarbitone (50mg/kg) as anaesthetic instead of urethane. In this test, the compounds of Examples 1, 2 and 3 have $ED_{50}$ values within the range 0.014 - 0.05 mg/kg.

In general, the compounds of the invention show no adverse effects at doses at which they effectively inhibit gastric acid secretion. Thus, for example, the compounds of Examples 1, 2 and 3 produced no toxic effects when administered intravenously to anaesthetised rats at a dose of at least 5 times their $ED_{50}$ values.

CLAIMS:

1. Compounds of the general formula (I)

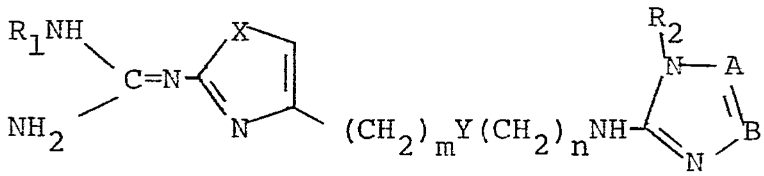

(I)

and physiologically acceptable salts and hydrates thereof, in which

$R_1$ represents a hydrogen atom or an alkyl, alkanoyl, aroyl or trifluoroalkyl group;

$R_2$ represents a hydrogen atom or an alkyl or alkenyl group or $C_{2-6}$ alkyl group substituted by a hydroxy or alkoxy group;

X represents a sulphur atom or NH;

Y represents an oxygen or sulphur atom or a bond;

m represents 1, 2 or 3;

n represents 2, 3 or 4;

A represents N and B represents $CR_3$; or

A represents $CR_3$ and B represents N; or

A and B each represent N; and

$R_3$ represents a hydroxy, hydroxyalkyl, alkoxyalkyl, acyloxyalkyl or CH=NOH group; or

$R_3$ represents the group $CH_2Z(CH_2)_rR_4$ in which r is 1 to 4 when Z is an oxygen atom, or r is 1 when Z is a sulphur atom, and $R_4$ is a $-CH_2OH$ group, the group $-CH=CH_2$, the group $-CH_2NR_5R_6$ where $R_5$ and $R_6$ are each alkyl or the group $-CH_2NHR_7$ where $R_7$ is alkyl; or

$R_3$ represents an aralkyl or heteroaralkyl group in which the alkyl portion is optionally substituted by a hydroxy or acyloxy group; or

$R_3$ represents the group $(CH_2)_qR_8$ where q is zero, 1, 2 or 3 and $R_8$ is the group $CH_2NHC(=D)NHR_9$ where D is NCN or $CHNO_2$ and $R_9$ is an alkyl group; or the group

$COR_{10}$ where $R_{10}$ is a hydrogen atom or a hydroxy, alkyl or $NR_{11}R_{12}$ group where $R_{11}$ and $R_{12}$ are the same or different and each represents a hydrogen atom or an alkyl group, or $NR_{11}R_{12}$ forms a 5 or 6 membered ring; or the group $SO_2R_{13}$ where $R_{13}$ is an alkyl or aryl group; or the group $NHSO_2R_{14}$ where $R_{14}$ is an alkyl group; or the group $NHCOR_{15}$ where $R_{15}$ is a hydrogen atom or an alkyl, alkoxy, aryl, heteroaryl or $NHR_{16}$ group where $R_{16}$ is an aryl, alkyl or cycloalkyl group; or

$R_3$ represents the group $CH_2SR_{17}$ where $R_{17}$ is an alkyl or heteroaryl group; or

$R_3$ represents a $C_{2-4}$ straight or branched alkyl group substituted by two hydroxyl groups or the dihydroxyalkyl group may form a cyclic ketal structure of the formula

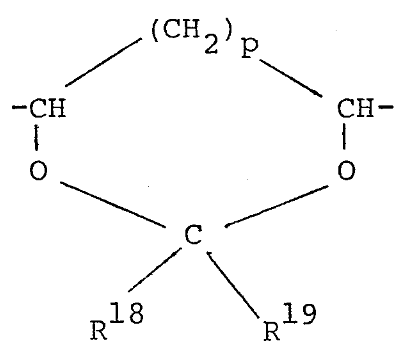

where p is zero or 1, and $R_{18}$ and $R_{19}$, which may be the same or different, each represents a $C_{1-4}$ alkyl group.

2. Compounds as claimed in claim 1, in which

$R_1$ is a hydrogen atom or a methyl, ethyl, propyl, isopropyl, butyl, acetyl, propionyl, benzoyl or 2,2,2-trifluoroethyl group; and

$R_2$ is a methyl, ethyl, propyl, allyl, 2-hydroxyethyl or 2-methoxyethyl group.

3. Compounds as claimed in claim 1 or 2, in which

$R_3$ is a group selected from hydroxy; hydroxymethyl; methoxymethyl; acetoxymethyl; formyloxymethyl;

benzoyloxymethyl; phenacetoxymethyl; -CH=NOH; $-CH_2Z(CH_2)_rR_4$ where $R_4$ is hydroxy, $-CH=CH_2$, di-$C_{1-3}$ alkylamino or $NHR_7$ where $R_7$ is $C_{1-3}$ alkyl; phenyl $C_{1-3}$ alkyl or heteroaryl $C_{1-3}$ alkyl in which the alkyl portion in each is unsubstituted or substituted by -OH or $C_{1-4}$ alkanoyloxy; methylthiomethyl; $(CH_2)_qR_8$, where $R_8$ is $CH_2NHC(=D)NHR_9$ (where D is NCN or $CHNO_2$ and $R_9$ is methyl); CHO; $-CO_2H$; alkoxycarbonyl; $-CONH_2$; $-CONHCH_3$; $-CON(CH_3)_2$; $CONR_{11}R_{12}$ (where $NR_{11}R_{12}$ is pyrrolidino); $SO_2R_{13}$ where $R_{13}$ is methyl or phenyl; $-NHSO_2CH_3$; NHCHO; $NHCOR_{15}$ where $R_{15}$ is methyl, ethoxy or phenyl; $NHCONHR_{16}$ where $R_{16}$ is phenyl, methyl or $C_{3-8}$ cycloalkyl; or 2,2-di-$C_{1-3}$ alkyl-1,3-dioxolan-4-yl.

4. Compounds as claimed in any of claims 1 to 3 in which the chain $-(CH_2)_mY(CH_2)_n-$ is $-CH_2S(CH_2)_2-$, $-CH_2O(CH_2)_3$ or $-(CH_2)_4-$.

5. Compounds as claimed in claim 1 in which:

$R_1$ is a hydrogen atom;

$R_2$ is a hydrogen atom, a $C_{1-4}$ alkyl group or a $C_{2-6}$ alkyl group substituted by a hydroxy group;

X is a sulphur atom;

Y is a sulphur atom;

m is 1;

n is 2;

$R_3$ is a hydroxy $C_{1-3}$ alkyl group.

6. N-[4-[[[2-[[3-(Hydroxymethyl)-1-methyl-1*H*-1,2,4-triazol-5-yl]amino]ethyl]thio]methyl]-2-thiazolyl] guanidine

and physiologically acceptable salts thereof; and

N-[4-[[[2-[(1-methyl-1*H*-tetrazol-5-yl)amino]ethyl] thio]methyl]-2-thiazolyl]guanidine

and physiologically acceptable salts thereof; and

N-[4-[[[2-[[1-(2-hydroxyethyl)-1H-tetrazol-5-yl]amino]ethyl]thio]methyl]-2-thiazolyl]guanidine and physiologically acceptable salts thereof.

7. A process for the preparation of compounds of formula (I) as defined in claim 1, which comprises:

a) for the preparation of compounds in which A or B is $-CR_3$ and in which $R_3$ is other than a dihydroxyalkyl group forming a cyclic acetal or cyclic ketal structure, or an aralkyl or heteroaralkyl group in which the alkyl portion is substituted by an acyloxy group, or acyloxyalkyl, CH=NOH, $SO_2R_{13}$, $COR_{10}$ (where $R_{10}$ is hydrogen or alkyl), or the group $(CH_2)_qR_8$, where $R_8$ is $CH_2NHC(=D)NHR_9$, cyclising a compound of formula (II)

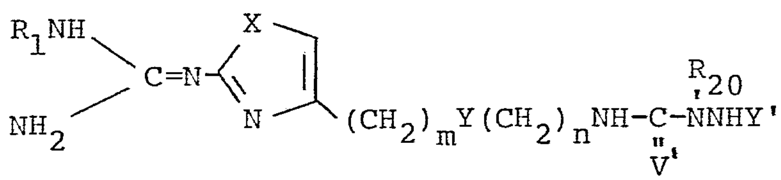

(II)

in which $R_{20}$ is a group as defined for $R_2$, V' is $N\underset{\overset{\|}{V}}{C}R_3^{a}$ and Y' is hydrogen where V is oxygen or sulphur and $R_3^{a}$ is a group as defined for $R_3$ or a group convertible thereto under the conditions of the cyclisation reaction; or V' is $NR_2$, $R_{20}$ is hydrogen and Y' is $\underset{\overset{\|}{Y''}}{C}R_3^{a}$ where Y" is oxygen or sulphur; or

b) for the preparation of compounds in which A and B both represent N, reacting an amine of formula (IV)

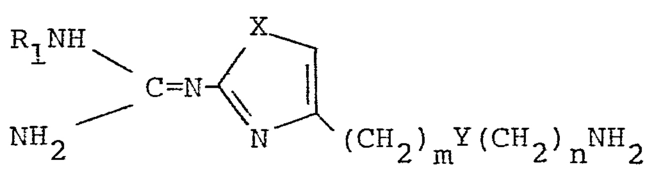

(IV)

optionally in the form of a salt, with a tetrazole of formula (V)

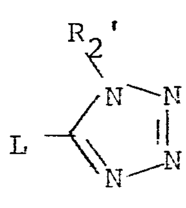

(V)

where L is a leaving group and $R_2'$ is the group $R_2$ or a group convertible thereto; or

c) for the preparation of compounds in which $R_3$ is the group $(CH_2)_qR_8$ in which $R_8$ is $COR_{10}$ where $R_{10}$ is hydrogen or alkyl, or $SO_2R_{13}$, oxidising the corresponding compound in which $R_3$ is the group $(CH_2)_qCHR_{10}OH$ or $(CH_2)_qSR_{13}$; or

d) for the preparation of compounds in which $R_3$ is CH=NOH, reacting the corresponding aldehyde with hydroxylamine; or

e) for the preparation of compounds in which $R_3$ is acyloxyalkyl, or $R_3$ represents an aralkyl or heteroaralkyl group in which the alkyl portion is substituted by an acyloxy group, reacting the corresponding alcohol with an activated derivative of an appropriate acid; or

f) for the preparation of compounds in which $R_3$ includes the group 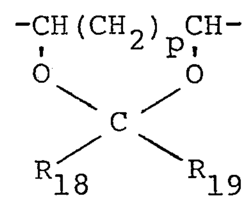

reacting a corresponding compound in which $R_3$ includes the group 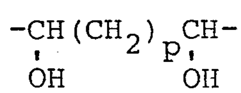

with a ketone $R_{18}R_{19}CO$ in the presence of an acid; or

g) for the preparation of compounds in which $R_3$ represents the group $CH_2NHC(=D)NHR_9$, treating the corresponding amino-alkyltriazole in which $R_3$ is the group $(CH_2)_{q+1}NH_2$ with a compound of formula $LC(=D)NHR_9$, where L is a leaving group;

and where the compound is formed in the form of a free base, optionally converting the free base into a salt.

8. A pharmaceutical composition comprising a compound as claimed in any of claims 1 to 6 together with at least one inert pharmaceutically acceptable carrier or diluent, optionally together with at least one other active ingredient.

9. Compounds of the general formula (V)

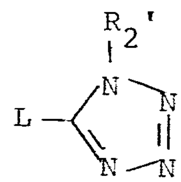

(V)

in which L is a leaving group and $R_2'$ is the group $CH_2CH_2OR_{20}$ where $R_{20}$ is a hydrogen atom or a hydroxyl protecting group.

CLAIMS: (AUSTRIA)

1. A process for the preparation of compounds of the general formula (I)

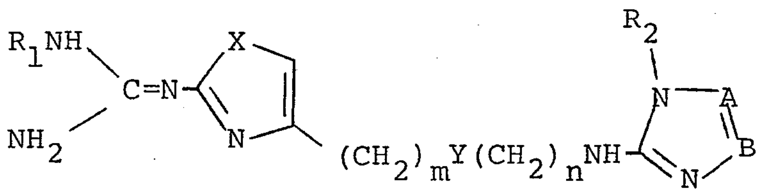

(I)

and physiologically acceptable salts and hydrates thereof, in which

$R_1$ represents a hydrogen atom or an alkyl, alkanoyl, aroyl or trifluoroalkyl group;

$R_2$ represents a hydrogen atom or an alkyl or alkenyl group or $C_{2-6}$ alkyl group substituted by a hydroxy or alkoxy group;

X represents a sulphur atom or NH;

Y represents an oxygen or sulphur atom or a bond;

m represents 1, 2 or 3,;

n represents 2, 3 or 4;

A represents N and B represents $CR_3$; or

A represents $CR_3$ and B represents N, or

A and B each represent N; and

$R_3$ represents a hydroxy, hydroxyalkyl, alkoxyalkyl, acyloxyalkyl or CH=NOH group; or

$R_3$ represents the group $CH_2Z(CH_2)_rR_4$ in which r is 1 to 4 when Z is an oxygen atom, or r is 1 when Z is a sulphur atom, and $R_4$ is a $-CH_2OH$ group, the group $-CH=CH_2$, the group $-CH_2NR_5R_6$ where $R_5$ and $R_6$ are each alkyl or the group $-CH_2NHR_7$ where $R_7$ is alkyl; or

$R_3$ represents an aralkyl or heteroaralkyl group in which the alkyl portion is optionally substituted by a hydroxy or acyloxy group; or

$R_3$ represents the group $(CH_2)_qR_8$ where q is zero, 1, 2 or 3 and $R_8$ is the group $CH_2NHC(=D)NHR_9$ where D is NCN or $CHNO_2$ and $R_9$ is an alkyl group; or the group

$COR_{10}$ where $R_{10}$ is a hydrogen atom or a hydroxy, alkyl or $NR_{11}R_{12}$ group where $R_{11}$ and $R_{12}$ are the same or different and each represents a hydrogen atom or an alkyl group, or $NR_{11}R_{12}$ forms a 5 or 6 membered ring; or the group $SO_2R_{13}$ where $R_{13}$ is an alkyl or aryl group; or the group $NHSO_2R_{14}$ where $R_{14}$ is an alkyl group, or the group $NHCOR_{15}$ where $R_{15}$ is a hydrogen atom or an alkyl, alkoxy, aryl, heteroaryl or $NHR_{16}$ group where $R_{16}$ is an aryl, alkyl or cycloalkyl group; or

$R_3$ represents the group $CH_2SR_{17}$ where $R_{17}$ is an alkyl or heteroaryl group; or

$R_3$ represents a $C_{2-4}$ straight or branched alkyl group substituted by two hydroxyl groups or the dihydroxyalkyl group may form a cyclic ketal structure of the formula

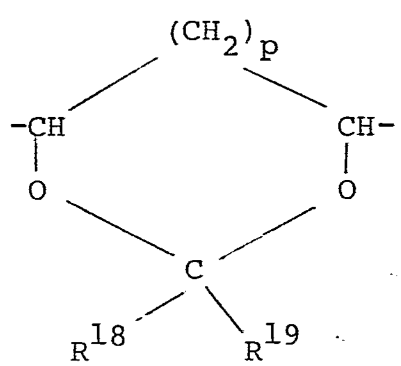

where p is zero or 1, and $R_{18}$ and $R_{19}$, which may be the same or different, each represents a $C_{1-4}$ alkyl group; which comprises

a) for the preparation of compounds in which A or B is $-CR_3$ and in which $R_3$ is other than a dihydroxyalkyl group forming a cyclic acetal or cyclic ketal structure, or an aralkyl or heteroaralkyl group in which the alkyl portion is substituted by an acyloxy group, or acyloxyalkyl, CH=NOH, $SO_2R_{13}$, $COR_{10}$ (where $R_{10}$ is hydrogen or alkyl ), or the group $(CH_2)_qR_8$, where $R_8$ is $CH_2NHC(=D)NHR_9$, cyclising a compound of formula (II)

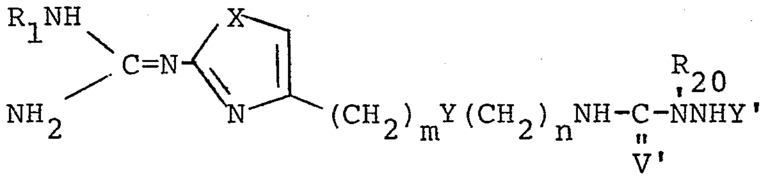

(II)

in which $R_{20}$ is a group as defined for $R_2$, V' is $N\underset{\underset{V}{\parallel}}{C}R_3{}^a$ and Y' is hydrogen where V is oxygen or sulphur and $R_3{}^a$ is a group as defined for $R_3$ or a group convertible thereto under the conditions of the cyclisation reaction; or V' is $NR_2$, $R_{20}$ is hydrogen and Y' is $\underset{\underset{Y''}{\parallel}}{C}R_3{}^a$ where Y" is oxygen or sulphur; or

b) for the preparation of compounds in which A and B both represent N, reacting a amine of formula (IV)

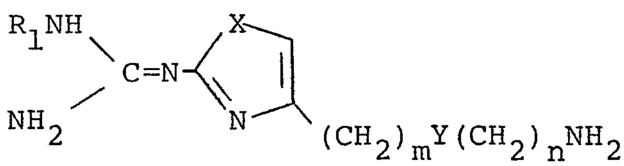

(IV)

optionally in the form of a salt, with a tetrazole of formula (V)

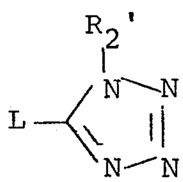

(V)

where L is a leaving group and $R_2$' is the group $R_2$ or a group convertible thereto; or

c) for the preparation of compounds in which $R_3$ is the group $(CH_2)_qR_8$ in which $R_8$ is $COR_{10}$ where $R_{10}$ is hydrogen or alkyl, or $SO_2R_{13}$, oxidising the corresponding compound in which $R_3$ is the group $(CH_2)_qCHR_{10}OH$ or $(CH_2)_qSR_{13}$; or

d) for the preparation of compounds in which $R_3$ is CH=NOH, reacting the corresponding aldehyde with hydroxylamine; or

e) for the preparation of compounds in which $R_3$ is

acyloxyalkyl, or $R_3$ represents an aralkyl or heteroaralkyl group in which the alkyl portion is substituted by an acyloxy group, reacting the corresponding alcohol with an activated derivative of an appropriate acid; or

f) for the preparation of compounds in which $R_3$ includes the group 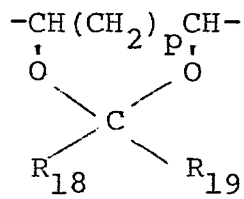

reacting a corresponding compound in which $R_3$ includes the group 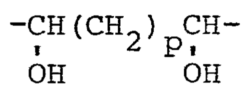

with a ketone $R_{18}R_{19}CO$ in the presence of an acid; or

g) for the preparation of compounds in which $R_3$ represents the group $CH_2NHC(=D)NHR_9$, treating the corresponding aminoalkyltriazole in which $R_3$ is the group $(CH_2)_{q+1}NH_2$ with a compound of formula $LC(=D)NHR_9$, where L is a leaving group;

and where the compound is formed in the form of a free base, optionally converting the free base into a salt.

2. A process as claimed in claim 1, for the preparation of compounds in which

$R_1$ is a hydrogen atom or a methyl, ethyl, propyl, isopropyl, butyl, acetyl, propionyl, benzoyl or 2,2,2-trifluoroethyl group; and

$R_2$ is a methyl, ethyl, propyl, allyl, 2-hydroxyethyl or 2-methoxyethyl group.

3. A process as claimed in claim 1 or 2, for the preparation of compounds in which

$R_3$ is a group selected from hydroxy; hydroxymethyl; methoxymethyl; acetoxymethyl; formyloxymethyl; benzoyloxymethyl, phenacetoxymethyl; $-CH=NOH$; $-CH_2Z(CH_2)_rR_4$ where $R_4$ is hydroxy, $-CH=CH_2$, di-$C_{1-3}$ alkylamino or $NHR_7$ where $R_7$ is $C_{1-3}$

alkyl; phenyl $C_{1-3}$ alkyl or heteroaryl $C_{1-3}$ alkyl in which the alkyl portion in each is unsubstituted or substituted by -OH or $C_{1-4}$ alkanoyloxy; methylthiomethyl; $(CH_2)_qR_8$, where $R_8$ is $CH_2NHC(=D)NHR_9$ (where D is NCN or $CHNO_2$ and $R_9$ is methyl); CHO; $-CO_2H$; alkoxycarbonyl; $-CONH_2$; $-CONHCH_3$; $-CON(CH_3)_2$; $CONR_{11}R_{12}$ (where $NR_{11}R_{12}$ is pyrrolidino); $SO_2R_{13}$ where $R_{13}$ is methyl or phenyl; $-NHSO_2CH_3$; NHCHO; $NHCOR_{15}$ where $R_{15}$ is methyl, ethoxy or phenyl; $NHCONHR_{16}$ where $R_{16}$ is phenyl, methyl or $C_{3-8}$ cycloalkyl; or 2,2-di-$C_{1-3}$ alkyl-1,3-dioxolan-4-yl.

4. A process as claimed in any of claims 1 to 3 for the preparation of compounds in which the chain $-(CH_2)_mY(CH_2)_n-$ is $-CH_2S(CH_2)_2-$, $-CH_2O(CH_2)_3$ or $-(CH_2)_4-$.

5. A process as claimed in claim 1 for the preparation of compounds in which:

$R_1$ is a hydrogen atom;

$R_2$ is a hydrogen atom, a $C_{1-4}$ alkyl group; or a $C_{2-6}$ alkyl group substituted by a hydroxy group;

X is a sulphur atom;

Y is a sulphur atom;

m is 1;

n is 2;

$R_3$ is a hydroxy $C_{1-3}$ alkyl group.

6. A process as claimed in claim 1 for the preparation of the compounds

N-[4-[[[2-[[3-(Hydroxymethyl)-1-methyl-1*H*-1,2,4-triazole-5-yl]amino]ethyl]thio]methyl]-2-thiazolyl] guanidine

and physiologically acceptable salts thereof;

N-[4-[[[2-[(1-methyl-1*H*-tetrazol-5-yl)amino]ethyl]

thio]methyl]-2-thiazolyl]guanidine
and physiologically acceptable salts thereof; and

N-[4-[[[2-[[1-(2-hydroxyethyl)-1*H*-tetrazol-5-yl]amino]ethyl]thio]methyl]-2-thiazolyl]guanidine
and physiologically acceptable salts thereof.

European Patent Office

# EUROPEAN SEARCH REPORT

0117368

Application number

EP 83 30 8037

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| P,A | CHEMICAL ABSTRACTS, vol. 99, no. 25, 19th December 1983, page 646, no. 212527w, Columbus, Ohio, US & SU - A - 1 030 361 (SCIENTIFIC-RESEARCH INSTITUTE OF PHYSICAL-CHEMICAL PROBLEMS MINSK) 23-07-1983<br>----- | | C 07 D 417/12<br>C 07 D 403/12<br>C 07 D 257/04<br>C 07 D 405/12<br>A 61 K 31/33 |
| | | | **TECHNICAL FIELDS SEARCHED (Int. Cl. 3)** |
| | | | C 07 D 417/00<br>C 07 D 403/00<br>C 07 D 257/00<br>C 07 D 405/00<br>A 61 K 31/00 |

The present search report has been drawn up for all claims

| Place of search | Date of completion of the search | Examiner |
|---|---|---|
| THE HAGUE | 04-05-1984 | DE BUYSER I.A.F. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document